# EUROPEAN PATENT APPLICATION

(11) **EP 3 960 164 A1**
(43) Date of publication of application: **02.03.2022**
(21) Application number: 20795882.8
(22) Date of filing: 21.04.2020
(51) Int. Cl.: A61K 9/28, A61K 9/44, A61K 9/48, A61J 3/06

(54) **METHOD FOR PRODUCING PHARMACEUTICAL FORMULATION**

(30) Priority: 22.04.2019 JP 2019081372
(71) Applicant: Nipro Corporation, Osaka-shi, Osaka 531-8510 (JP)
(72) Inventor: NAKANO Yoshio, Osaka-shi, Osaka 531-8510 (JP); HAYASHIDA Tomohiro, Osaka-shi, Osaka 531-8510 (JP); HOASHI Yohei, Osaka-shi, Osaka 531-8510 (JP); EDAMURA Keisuke, Osaka-shi, Osaka 531-8510 (JP); ICHIKAWA Yuki, Osaka-shi, Osaka 531-8510 (JP)
(74) Representative: Reitstötter Kinzebach
(86) International application number: PCT/JP2020/017254
(87) International publication number: WO 2020/218310

(57) **Abstract**

[Problem to be Solved]

Provided is a method for manufacturing a pharmaceutical preparation, which safely and simply enables uniformization of contents of a small amount of a drug.

[Solution]

The method for manufacturing the pharmaceutical preparation includes: using a liquid for dispensing, the liquid containing0 a drug; and a first application step of applying the drug to a surface of a tablet or a capsule by a dispensing method. In addition, a pharmaceutical preparation according to the present invention includes: a base; and a drug which is applied to a surface of the base by a dispensing method, and the base is a tablet or a capsule.

## Description

### TECHNICAL FIELD

The present invention relates generally to a method for manufacturing a pharmaceutical preparation and, in particular, to a method for manufacturing a pharmaceutical preparation having a small drug content.

### BACKGROUND ART

Among dosage forms of a pharmaceutical preparation, tablets and capsules are manufactured by compression-molding a mixture, which contains a medicinal component (hereinafter, also referred to as a "drug") and various additives, into round forms, disk forms, elliptical forms, circular forms, or the like or by filling the mixture in the round forms, the disk forms, the elliptical forms, the circular forms, or the like. Since the tablets and the capsules have many advantages such as ease of handling and being large-scale producible, the tablets and the capsules are dosage forms which are most heavily used as solid preparations for oral administration.

The tablets or the capsules are ordinarily manufactured by selecting an appropriate manufacturing method from a direct powder compression method or a filling method; a dry granule compression method or a filling method; a wet granulation granule compression method or a filling method; or the like depending on physical properties of the medicinal component and the additives. In any of the manufacturing methods, the medicinal component and various additives are sieved; thereafter, the sieved medicinal component and additives are subjected to manipulations such as mixing, granulation, and drying; and granules are prepared to be provided for subjecting the granules to a tableting or filling process. In a mixing manipulation process, many of the medicinal components are subjected to excipient dilution work or the like in order to fill a predetermined amount in accordance with a component content in one piece of the preparation.

Since a required dosage amount of a medicinal component having high activity is small, an amount contained in a tablet or a capsule is extremely small. Specifically, for example, as a drug having high bioactivity, whose dosage amount per administration is small, there are a steroidal hormone drug such as ethinylestradiol, active vitamin Ds used as therapeutic agents for osteoporosis, and the like. A dosage amount per administration of each of these drugs is one mg or less, and it is required for a drug content per one tablet or one capsule to be extremely small.

It is difficult to manufacture the preparation as an excipient-diluted agent by directly mixing the above-mentioned small amount of the medicinal component and pulverulent bodies together with various additives. Therefore, variation in contents of the medicinal component among the tablets or the capsules is easily caused.

It is extremely difficult to suppress the variation of the medicinal component in the tablets or the capsules and ensure content uniformity in manufacturing in which a scale is ordinarily large (200 kg to 300 kg). In addition, when the scale is large, it is likely that various situations such as segregation or variation of the contents, a loss due to adherence to a machine wall, and the like are caused, strict management in manufacturing processes is required, and it is often the case that a bottleneck in formulation arises.

Furthermore, in a case of the tablets, also in view of disintegration properties in a body, elution properties of the medicinal component, and bioavailability, appropriate additives and manufacturing method are selected, and it is required for contents to be uniform.

Furthermore, in a case where the medicinal component has strong hygroscopic properties or adhesive properties or a case where a combination of the medicinal component and the additives is bad, it is further difficult to maintain contents of the medicinal component in the tablets constant, causing poor yield and stability.

Therefore, conventionally, as a method for inputting a small amount of an active substance, that is, a drug into tablets or capsules without impairing uniformity, there is a method in which the drug is perseveringly dispersed while the drug is evenly diluted with other excipient and the like.

For example, described in Japanese Patent Application Laid-Open Publication No. 2006-06303 (Patent Literature 1) is a method for manufacturing tablets which is characterized in that a solution containing a medicinal component is wholly and evenly added to an excipient under stirring by dropping or spraying; sufficient blending is conducted; thereafter, drying is conducted; powder is obtained by adding granulated lactose to the dried powder and evenly blending the resultant to obtain powder; and the obtained powder is molded. Each of the tablets obtained in the above-described method contains an extremely small amount of the medicinal component, whose judged value in a content uniformity test prescribed in The Japanese Pharmacopoeia Fourteenth Edition is less than 15%.

As described above, in the conventional manufacturing processes, as the method employed to ensure the content uniformity in manufacturing tablets (tableting) or manufacturing capsules (filling), a method in which some processes in the manufacturing processes of the tablets or the capsules are elaborated, a blending process, an excipient-dilution process, and a sieving process are combined, and uniformity in a blended pulverulent body is made as high as possible is a general method for manufacturing.

In addition, for example, there has been proposed in U.S. Pat. No. 4322449 (Patent Literature 2) a method in which a liquid or a dispersion liquid containing an active component is dotted on a base such as a pulverulent body for tableting or filling into capsules by using a piezoelectric dosing system. It is described in Examples in Patent Literature 2 that a water-ethanol solution of a drug is placed onto the base.

### CITATION LIST

### PATENT LITERATURE

Patent Literature 1: Japanese Patent Application Laid-Open Publication No. 2006-063030
Patent Literature 2: U.S. Pat. No. 4322449

### SUMMARY OF THE INVENTION

### TECHNICAL PROBLEM

However, in the method described in Patent Literature 1, big problems in the manufacturing processes and the analysis processes in that in the manufacturing processes, strict process management for uniformly dispersing the medicinal component in the tablets is required, in that sampling and analysis of the granules and the pulverulent body are extremely delicate, and in other respect are present.

In addition, many of the drugs applied as small-amount contained drug products have strong drug efficacy in general and thus also have strong toxicity, and when such drug is tableted, it is required to pay attention to aspects of hardware and software in handling thereof. However, in the conventional method described in Patent Literature 1, manipulations of workers intervene in the processes, and there is a danger that the workers are exposed to the drug.

As measures indicating strengths of the efficacy and the toxicity of the drug, there are occupational exposure limits (OEL) (International Society for Pharmaceutical Engineering, Inc. Japan Affiliate "Performance Evaluation of Particle Containment of Drug Manufacturing Apparatuses" in 2007). Each of these measures is indicated by an amount (µg/m³) of a drug, and the amount is an amount in an environment which is safe for workers even in a case where the workers in that concentration environment work and are exposed thereto for eight hours per day. There are categories 1 to 6 of the OEL. In accordance with an increase in the number of the categories, each of the drug efficacy and the toxicity becomes strong, and it is required to make working under an environment in which a low concentration or less is maintained. For example, it is only required for a drug of a category 4 to maintain an amount of 10 µg/m³ or less, and a manufacturing facility in which such an environment is maintained is needed.

In a case where a high active component (with the category 4 or more of the OEL) is tableted as a drug, in order to prevent diffusion of the drug and to suppress exposure of workers as much as possible, confinement (containment) is conducted. It is required for the containment to arrange a facility for a highly active drug (hardware) to prevent diffusion of power dust of the drug and the like to the outside by making a working room a negative pressure environment and further, to arrange management of software for a working method of the workers. In the method described in Patent Literature 1, the containment is conducted, and moreover, in addition to processes such as the blending process of powder, the tableting process, and the capsule filling process in the method for manufacturing, in order to enhance uniformity, not only a special manufacturing facility for the highly active component is required, but also it is required to adopt extra processes such as an excipient dilution process accompanied with cumbersome manipulations.

Furthermore, as to the facility for the highly active drug, as compared with the conventional manufacturing facility, many items such as management of air conditioning and the like of HVAC, multiplexing of a working room (manipulations inside isolators), balance between a space size and a number of times of ventilation, facility washability, and the like are complex, and due to a cost for building the facility itself and complication of workability, a formulation cost is inevitably increased.

In addition, also in manufacturing using equipment within the facility for the highly active drug, only machines and processes which suppress scattering of the drug can be adopted, and there may be a case where the manufacturing method is greatly limited.

On the other hand, in the method described in Patent Literature 2, droplets of a comparatively large amount of a drug liquid, whose number is considerably large, are sprayed in a finely dotted manner by using spraying nozzles whose number is small and are thereby placed onto a carrier. Therefore, in a case of a drug whose content is small, contents cannot be accurately made uniform. In addition, although it is described in Examples in Patent Literature 2 that a water-ethanol liquid of the drug is sprayed to a base, a viscosity of a solvent is insufficient, and there is a fear that after scattering the solvent, the drug is exfoliated.

Therefore, an object of the present invention is to provide a method for manufacturing a pharmaceutical preparation, which safely and simply enables uniformization of contents of a small amount of a drug.

### SOLUTION TO PROBLEM

The present inventors, et al. have devoted themselves to studies as to a method for manufacturing tablets or capsules, each of which contains a small amount of a drug whose bioactivity is high in a safe and simple manner, that is, a manufacturing method in which a fear of exposure of workers to the drug is small and contents are made uniform by simple manufacturing processes.

As a result, it was found out that by employing a dispensing method rapidly developing in recent years, a solution or a dispersion liquid, which contains a drug, is applied to tablets or capsules, thereby allowing the tablets or the capsules, each of which contains a small amount of the drug, to be manufactured simply and moreover, accurately in uniformity of contents.

The dispensing method is a kind of a method for application and is a method in which a constant amount of an application substance, whose amount is small, is applied by using an apparatus in a contacting or contactless manner. Conventionally, the dispensing method is employed to apply an adhesive or the like when small-sized electronic components are manufactured.

In other words, a drug is applied to surfaces of tablets or capsules by the dispensing method, thereby allowing uniformity of the tablets or capsules, each of which contains a small amount of the drug, to be enhanced and furthermore, the tablets or capsules, each of which contains a small amount of the drug, to be efficiently and safely manufactured.

Based on the above-described findings, the present invention is configured as described below.

A method for manufacturing a pharmaceutical preparation according to the present invention includes: using a liquid for dispensing, the liquid containing a drug; and a first application step of applying the drug to a surface of a tablet or a capsule by a dispensing method.

In the method for manufacturing the pharmaceutical preparation according to the present invention, it is preferable that the first application step is a process in which the drug is printed by ink jet printing as the dispensing method.

In the method for manufacturing the pharmaceutical preparation according to the present invention, it is preferable that a coating step of coating the drug applied in the first application step is further included.

In the method for manufacturing the pharmaceutical preparation according to the present invention, it is preferable that an amount of the drug applied in the first application step is 15 mg or less.

In the method for manufacturing the pharmaceutical preparation according to the present invention, it is preferable that a second application step of applying a second drug or an excipient to a surface of a tablet or a capsule by a dispensing method is further included, the second drug being different from the drug applied in the first application step, and the second drug or the excipient applied in the second application step is printed in such a way as to be separated from the drug applied in the first application step.

In the method for manufacturing the pharmaceutical preparation according to the present invention, it is preferable that the second application step is a process in which the second drug or the excipient is printed by ink jet printing as the dispensing method.

In the method for manufacturing the pharmaceutical preparation according to the present invention, it is preferable that the first application step is conducted in a closed system.

In the method for manufacturing the pharmaceutical preparation according to the present invention, it is preferable that an application area of the drug applied in the first application step is determined based on an amount of the drug contained in the pharmaceutical preparation.

In the method for manufacturing the pharmaceutical preparation according to the present invention, it is preferable that relative standard deviation (RSD) of a content of the drug applied in the first application step is less than 3.8%.

In the method for manufacturing the pharmaceutical preparation according to the present invention, it is preferable that a category of occupational exposure limits (OEL) of the drug applied in the first application step or the second drug applied in the second application step is 4 or more.

A method for uniformizing a drug content in a pharmaceutical preparation includes a step of manufacturing the pharmaceutical preparation by the method for manufacturing the pharmaceutical preparation according to any one of the above-mentioned methods for manufacturing the pharmaceutical preparation.

A method for preventing scattering of a drug in manufacturing of a pharmaceutical preparation includes a step of manufacturing the pharmaceutical preparation by the methods for manufacturing the pharmaceutical preparation according to any one of the above-mentioned methods for manufacturing the pharmaceutical preparation.

A pharmaceutical preparation includes: a base; and a drug applied to a surface of the base by a dispensing method, and the base is a tablet or a capsule.

In the pharmaceutical preparation according to the present invention, it is preferable that the drug is applied to the surface of the base together with a binder by the dispensing method.

In the pharmaceutical preparation according to the present invention, it is preferable that the drug is a drug printed by ink jet printing as the dispensing method.

In the pharmaceutical preparation according to the present invention, it is preferable that coating which coats the drug applied by the dispensing method is further included.

In the pharmaceutical preparation according to the present invention, it is preferable that an amount of the drug applied by the dispensing method is 15 mg or less.

In the pharmaceutical preparation according to the present invention, it is preferable that a second drug or an excipient is included, the second drug applied to a surface of the base by a dispensing method in such a way as to be separated from the drug applied by the dispensing method, the second drug being different from the drug.

In the pharmaceutical preparation according to the present invention, it is preferable that the second drug or the excipient is a drug printed by ink jet printing as the dispensing method.

In the pharmaceutical preparation according to the present invention, it is preferable that relative standard deviation (RSD) of a content of the drug applied by the dispensing method is less than 3.8%.

In the pharmaceutical preparation according to the present invention, it is preferable that each of categories and/or a category of occupational exposure limits (OEL) of the drug and/or the second drug is 4 or more.

### BRIEF DESCRIPTION OF THE DRAWINGS

FIG. 1 show photographs of tablets in Examples 1 to 4.
FIG. 2 show photographs of tablets in Examples 5 to 7.
FIG. 3 is a graph showing relationship between contents of a drug and printing areas of the drug of the tablets in Examples 1 to 7.
FIG. 4 is a graph showing relationship between drug contents and residual rates in Examples 14 to 15 and Comparative Example 5.

### DESCRIPTION OF EMBODIMENT

Hereinafter, an embodiment of the present invention will be described. Note that in description given below, a concentration (%) means % by weight unless otherwise specified.

A method for manufacturing a pharmaceutical preparation according to the present invention includes a first application step of applying a drug to a surface of a tablet or a capsule by using a liquid for dispensing which contains the drug or preferably a liquid for dispensing which contains the drug and a binder and by employing a dispensing method. The pharmaceutical preparation manufactured according to the present invention contains a base and the drug applied by the dispensing method on a surface of the base, and the base is a tablet or a capsule. In the pharmaceutical preparation, preferably, the drug is applied onto the surface of the base together with the binder by the dispensing method.

The base to which the drug is applied by the dispensing method of the present invention is the tablet or the capsule. The tablet may be a conventional tablet (a raw tablet or a film-coated tablet) or may be an orally disintegrating tablet. However, the tablet which has hygroscopic properties (solvent absorbing properties) which do not allow the surface of the tablet to be excessively wet by the liquid for dispensing such as the drug containing liquid (dispersion liquid) and the excipient containing liquid (dispersion liquid) is preferable. The capsule may be a hard capsule or a soft capsule.

As the dispensing method, there are various methods depending on objects or discharge methods. For example, there are a pneumatic (syringe) method, a capacity measuring method, a non-contact method (a pneumatic method, a piezoelectric method, or the like), a tubing method, a plunger method, and the like. These are technologies which are applied to manufacturing in relation to electronic components, automobiles, displays, batteries, and the like. In a pharmaceutical field, the dispensing method is used in an experiment aiding manner in which a reagent is dispensed or applied.

In relation to the electronic components and the like, many of the liquids for dispensing in fields to which the conventional dispensing method is applied are industrial materials such as a minute amount of an adhesive, a coating agent, a sealing agent, and grease. However, in the field of the present invention, the liquid for dispensing is a solution containing a drug or a scattering suspension and is easily physically manipulated, as compared with the above-mentioned industrial materials.

The application of the drug to the tablet by the dispensing method is particularly suitable in a case where an application amount is several pL to several tens µL. Among the dispensing methods, in the later-described ink jet printing, application of the drug to the tablet by the dispensing method is particularly suitable in a case where an application amount is several pL to several tens nL.

### [Drug]

The drug which is applicable in the present invention is not particularly limited as long as the drug is a medicinal active component which is effective in therapeutics or preventive medicine. In order to contain a drug whose each one dosage amount is 15 mg or less or further, 10 mg or less in each of tablets in uniform contents, the method for manufacturing of the present invention is particularly preferable.

As the medicinal active component, for example, a steroid hormone drug such as male hormones, female hormones, and adrenocortical hormones or an active vitamin D drug which is a drug for osteoporosis is considered as a representative of a small-amount drug. Beside the above-mentioned drugs, the medicinal active component may be any drug as long as drug activity is high and a dosage amount is small, and beside the above-mentioned drugs, cited are a hypnotic sedative drug; a sleep inducer; a migraine drug; an anti-anxiety drug; an antispasmodic drug; an antidepressant drug; an anti-parkinson drug; a drug for psychoneurosis; a drug for a central nervous system; a local anesthetic; a skeletal muscle relaxant; an autonomic nerve drug; an antipyretic analgesic antiphlogistic; an antispasmodic drug; an anti-dizziness drug; a cardiotonic agent; an antiarrhythmic agent; an antihypertensive agent; a vaso-constricting drug; a vessel dilator; a drug for circulation organs; an agent for hyperlipidemia; an antitussive drug; an expectorant drug; an antitussive expectorant agent; a bronchodilator agent; an antidiarrheic drug; a drug for an urinary organ; a hemostatic agent; a drug for a liver disease; a gout treating drug; an antidiabetic agent; an antihistaminic drug; an antibiotic drug; an antimicrobial agent; an anti-malignant tumor drug; a chemotherapeutic agent; and the like.

Besides the above-mentioned drugs, for example, an antigenic substance (protein) of antigen-antibody reaction of an immune system or an adjuvant which boost immune reaction by a small amount of an antigen can also be applied to tablets, instead of each of the above-mentioned drugs. Although it is clear that the present invention can be applied to an ordinary small-amount containing drug, since in the dispensing method, a manipulation space can be saved and manipulations in a closed system is possible, the present invention can be applied to an environment having the category 4 or more of the occupational exposure limits (OEL) of the drug by arranging an environment where an apparatus is installed.

### [Liquid for Dispensing]

The liquid for dispensing of the present invention includes at least a drug. It is preferable that the liquid for dispensing includes a binder. Properties of the liquid for dispensing such as a viscosity, a solvent, and a liquid temperature are appropriately selected in accordance with an object and conditions.

The viscosity shows a degree of viscidity of a fluid (stickiness) and is represented by a unit of Pa·s (Pascal·second). The viscosity is measured by employing a viscosity determination method in conformity with the prescription "2. Method II Viscosity measurement by rotational viscometer in 2.53 Viscosity Determination in General Tests" in The Japanese Pharmacopoeia Seventeenth Edition and by using (2.1.2.) a single cylinder-type rotational viscometer (Brookfield type viscometer). Viscosity data of liquids used in the following Examples shows results measured at 20°C.

It is preferable that the viscosity of the liquid for dispensing is 0.5 mPa·s to 200 Pa·s, it is more preferable that the viscosity of the liquid for dispensing is 1 mPa·s to 100 Pa·s, it is further preferable that the viscosity of the liquid for dispensing is 2 mPa·s to 50 Pa·s, and it is still further preferable that the viscosity of the liquid for dispensing is approximately 5 mPa·s to 200 mPa·s. For example, as described in Examples in detail, in a case where as a dispenser, a product AERO JET manufactured by Musashi Engineering, Inc. is used, it is preferable that the viscosity of the liquid for dispensing is 1.0 mPa·s to 100 Pa·s, it is more preferable that the viscosity of the liquid for dispensing is 2.0 mPa·s to 50 Pa·s, and it is further preferable that the viscosity of the liquid for dispensing is 5 mPa·s to 200 mPa·s. In addition, in a case where as a dispensing method, ink jet printing is used, it is preferable that the viscosity of the liquid for dispensing is 0.5 mPa·s to 200 mPa·s, it is more preferable that the viscosity of the liquid for dispensing is 1 mPa·s to 100 mPa·s, it is further preferable that the viscosity of the liquid for dispensing is 2 mPa·s to 50 mPa·s, and it is still further preferable that the viscosity of the liquid for dispensing is approximately 5 mPa·s to 50 mPa·s. In a case where as the dispensing method, a method other than the ink jet printing is used, it is preferable that the viscosity of the liquid for dispensing is approximately 50 mPa·s to approximately 200 mPa·s.

As the solvent or a disperse medium for the liquid for dispensing, various solvent systems can be used. Accordingly, by appropriately adjusting an amount required for application, a viscosity, a solvent (disperse medium) system, optimum prescription for the liquid for dispensing can be selected.

A concentration of the drug in the liquid for dispensing is appropriately selected in accordance with an object and conditions. As the liquid for dispensing, various liquids from liquids whose each application drug amount is small to liquids whose each application drug amount is large and liquids whose each amount in a slurry state is large can be selected.

In a case where the drug dissolves in the solvent system, a drug solution can be used as the liquid for dispensing. In a case where the drug does not dissolve in the solvent system, the drug is suspended in the solvent system and a liquid obtained by blending the later-described film coating agent to increase a viscosity can be used as the liquid for dispensing.

As prescription of the liquid for dispensing, it is required to select a prescription system in which the drug is in a liquid state (even when the drug is dissolved in the solvent or is suspended and dispersed therein) and which exerts no influence such as instability for a period up to when spraying and applying is conducted, for example, prescription in which drug particles are not precipitated by a viscosity increaser.

Although conditions of the liquid for dispensing used in the ink jet printing in particular among the dispensing methods are specifically described later, in order to set a prescription system which satisfies prerequisites (a viscosity and surface tension) of the liquid for dispensing, (a) water and/or an organic solvent as a solvent are/is added, (b) a polymer (binder) to impart viscosity is added, and in some cases, (c) a plasticizing agent is added. Furthermore, in order to control the surface tension, (d) there may be a case where a surfactant agent is added.

As the organic solvent, lower alcohol such as ethanol, 1-propanol, 2-propanol, 1-butanol, and 2-butanol, propylene glycol, or polyethylene glycol (polyethylene glycol whose average molecular weight is 2,000 or less) is preferable, and among thesea , ethanol is most preferable. In a case where the water-soluble polymer in (b) with a low viscosity is used, it is preferable that a blending ratio of each of these organic solvents is 10% by weight to 80% by weight and it is more preferable that the blending ratio thereof is 15% by weight to 50% by weight. In addition, it is preferable that the polymer to impart the viscosity is water-soluble.

It is preferable that the liquid for dispensing includes a polymer generally used in ordinary film coating prescription of tablets as a binder. The liquid for dispensing includes the film coating agent, thereby allowing the drug to be fixed onto surfaces of tablets or capsules. As the film coating agent, for example, there are polymers such as a cellulose-based coating agent (hydroxypropylcellulose, hypromellose, hypromellose phthalate, or the like), polyvinyl alcohol, and copolyvidone. As an example of the water-soluble polymer used as the binder, more specifically, polyvinyl alcohol-acrylic acid- methyl methacrylate copolymer, hydroxypropyl cellulose, polyvinyl pyrrolidone, hydroxypropyl methyl cellulose, polyvinyl alcohol, a polyvinyl alcohol-polyethylene glycol graft copolymer, an ethyl acrylate-methyl methacrylate copolymer dispersion, or the like is preferable. Among these, in view of a viscosity and physical properties, the polyvinyl alcohol-acrylic acid- methyl methacrylate copolymer, the hydroxypropyl cellulose, the polyvinyl pyrrolidone, and the polyvinyl alcohol-polyethylene glycol graft copolymer are more preferable.

As a film coating agent other than these water-soluble polymers, naturally derived dextrin or polysaccharides (gum arabic) are also used as an alternative viscosity increaser (adhesion agent) and can be used as long as the same effect is exhibited.

Since (d) there are various grades in these water-soluble polymers and properties of the polymers are different from one another, accurate prescription by molecular weights cannot be made. In addition, as the above-mentioned dispersion medium, in order to lower the surface tension of the drug solution or the dispersion liquid, a surfactant agent may be added to a mixed liquid of water and lower alcohol as needed.

As the surfactant agent, sorbitan fatty acid ester (Polysorbate 80, NONION OP80-R, manufactured by NOF CORPORATION) or sucrose fatty acid ester (SURFHOPE S-1570, manufactured by Mistubishi-Chemical Foods), whose HLB value is approximately 15, is preferable. In addition, it is preferable that a used amount is less than 1%.

As (c) the plasticizing agent used in the present invention, for example, a plasticizing agent which dissolves or is miscible in the water or the lower alcohol is preferable, cited are sesame oil, castor oil, cotton oil, soybean oil, olive oil, canola oil, olein acid, a dimethylpolysiloxane-silicon dioxide mixture, medium-chain triglyceride, triethyl citrate, triacetin, diethyl phthalate, dibutyl phthalate, butyl-phthalyl-butyl glycolate, polyethylene glycol, and the like, and among these, the triethyl citrate and the dibutyl phthalate are preferable. Each of these plasticizing agents is effective as the plasticizing agent of the below-mentioned release control film-forming agent and enteric film-forming agent.

This plasticizing agent not only enhances film forming properties of the polymer but also serves to prevent clogging caused in a nozzle head part upon ink jets praying in a case where the drug does not sufficiently dissolve in a water- organic solvent system and is a drug liquid like pigmented ink, which is obtained by making the drug itself fine, and further, to prevent solidification and drying of a solid content during the manipulations.

In the dispensing method, liquids for dispensing having a significantly wide viscosity range can be used in general. It is preferable that a viscosity of the liquid for dispensing is 0.5 mPa·s to 200.0 Pa·s. In a case where as the dispensing method, the ink jet printing is used, it is preferable that the viscosity of the liquid for dispensing is adjusted to be within a range of 0.5 mPa·s to 200.0 mPa·s. In the ink jet printing, by employing an ink jet method with high accuracy, which has been developed in recent years, a liquid for dispensing which has a viscosity even up to 400 mPa·s can be applied.

Although these water-soluble polymers are used for combination and adhesion and as a dispersant, in accordance with an object of the drug, each of water dispersion liquids such as ethyl cellulose used as a release control film-forming agent; powder of an ethyl acrylate-methylmethacrylate-methacrylic acid chloridized trimethyla mmonium ethyl methacrylate copolymer; and ethyl cellulose; an ethyl acrylate-methylmethacrylate-methacrylic acid chloridized trimethyla mmonium ethyl methacrylate copolymer, and an ethyl acrylate-methylmethacrylate copolymer, each of which contains in the form of latex, is used, and slight sustained-release properties can alsobe imparted thereto.

In a case where the drug has a problem under acid conditions, there may be a case where hydroxypropyl methyl cellulose phthalate used as an enteric film-forming agent; hydroxypropyl methyl cellulose acetate succinate; a methacrylic acid-methylmethacrylate copolymer; a methacrylic acid-ethyl methacrylat copolymer; a methacrylic acid-ethyl acrylate copolymer; or the like is used. Needless to say, each of these is dissolved by using an organic solvent such as ethanol and isopropanol to be used, and it is also free to use a water dispersion liquid containing each of these water-insoluble polymers in the form of latex.

In addition, in a case where the drug has a bitter taste, for the purpose of masking the bitter taste, besides the release control film-forming agent and the enteric film-forming agent, polyvinyl acetal diethyl aminoacetate which is a gastrosoluble film-forming agent; a methylmethacrylate-methyl methacrylate-butyl methacrylate-dimethylaminoethyl methacrylate copolymer, or the like can be used.

### [Dispensing Apparatus]

Among dispensers (dispensing apparatuses) having various discharge methods, an appropriate dispenser is selected in accordance with an object and conditions. For example, in an application in which an application amount is large, in consideration of liquid exhaustion, a range and accuracy of discharge amounts, a degree of completion of a system which operates the dispenser, physical properties (a concentration, a viscosity, and a solvent) of a liquid, and the like, a jet type (contactless) dispenser is suitable. Depending on characteristics and a range of discharge liquid amounts of a liquid for dispensing, a syringe type dispenser, a capacity-measuring type dispenser, and the like are applicable, and among these, there is an apparatus which is set in an X-Y robot or the like to be used as needed.

In the dispenser, it is preferable that a material which does not cause any problem such as adsorption of a drug is used for a liquid contacting part which contacts the liquid for dispensing, that is, a drug-containing solution or a dispersion liquid. In addition, it is preferable that cleaning of the dispenser is easy.

In a system which discharges a constant amount of a liquid in an extremely small amount in the dispensing method, nozzles are used in general. Relationship between a nozzle diameter and a size of a drug which is suspended and dispersed in the liquid for dispensing is important. In view of an application amount of an object, prescription, and the like, the nozzle diameter is selected. As one example, a nozzle diameter of 19 G (0.69 mm) or 26 G (0.25 mm) can be used. A drug having an ordinary particle size of a several tens µm level can be discharged with the above-mentioned gauge diameter without causing any problems. In other words, it is not required to make particles of a drug fine in the ink jet method with high accuracy.

### [Ink Jet Printing]

In a case where as the dispensing method, the ink jet printing is used, as a dispenser, specifically, for example, any of currently generally used ink jet type tablet printing machines can be used. For example, IIM-4001 (manufactured by MATSUOKA MACHINERY WORKS CO., LTD), DP-i1100 or DP-i1300 (manufactured by SCREEN Holdings Co., Ltd), IMS-300D (manufactured by Qualicaps Co., Ltd.), APOLLO-2000KI (manufacture by Kyoto Seisakusho Co., Ltd.), TABREX Rev (manufactured by Freund Corp.), or the like is used, although the present invention is not limited thereto. Preferably, a model of a machine in which excessive force is not exerted on a conveyance path (a line of flow) of tablets is preferable.

Since in the ink jet printing apparatus used according to the present invention, instead of dye or pigment of ink, the drug is used, with attention paid to several points such as stability in the solution or the dispersion liquid and the like, in consideration of an ink jet type, cleanability of the machine, and the like, the ink jet printing apparatus is selected. Specifically, in a structure of the ink jet printing apparatus, it is preferable that a passing route of tablets or capsules and parts around the passing route have good cleanability upon supplying an applied liquid and replacing nozzles, that a number of portions which contact various kinds of ink (the drug or the active substance in the present invention) in parts of the machine which are replaced and cleaned is small, and that it is needed to clean only a small number of parts in the apparatus.

In addition, also as to a type of an ink jet type nozzle head part, a type thereof which has various new functions, not only a function of simple printing but also, for example, a function of accurately adjusting a spraying amount even in a case where a viscosity of a sprayed liquid is large, a function of coating tablet surfaces and other function, other than printing, and in which the above-mentioned functions can be easily equipped is preferable.

An ink discharge type of the ink jet printing is roughly divided into a continuous type and a drop-on-demand type. It is preferable that various printing processes in the present invention are performed by the drop-on-demand type which allows a needed amount of the ink to be jet-applied (ink jet-printed).

As an ink droplet discharge type in the drop-on-demand type, there are a thermal type and a piezo type. In the former, heat is partially instantaneously applied to the ink and in the latter, only a pressure is applied thereto. Accordingly, as the ink jet type used in the various printing processes in the present invention, in a case of a drug which is weak to the heat, the piezo type is preferable. However, in a case of the drug which is strong to the heat, the thermal type can also be applied.

As the prescription of the solution or the dispersion liquid which contains the drug used in the present invention, basically, as long as a solution or a dispersion liquid has the same physical properties (a viscosity, surface tension, and the like) as those of ink used in printing industry, any prescription can be applied to the method for manufacturing of the present invention. The prescription of the ink in the ink jet printing to surfaces of the tablets or the capsules is considered to be on extension of prescription of film coating of tablets in general. In the film prescription, in order to enhance combining properties to the tablets and dispersion properties of pigment such as a titanium oxide and iron oxides, a polymer such as hypromellose is used, and in order to enhance film forming properties thereof, a 10% to 30% plasticizing agent is added. As to prescription of the ink jet type sprayed drug solution or dispersion liquid, it is only required to prepare a solution or a dispersion liquid in consideration of such film coating and to apply the solution and dispersion liquid to the ink jet type.

As the ink, an edible ink to which an additive used as a fool additive is applied and an edible ink in which a raw material ordinarily used as a medicinal additive is used can be used.

As the ink applied to the ink jet printing apparatus in general, there are a dye type in which dye is dissolved and a pigment type in which pigment is dispersed. In a case of the edible dye or pigment, water, alcohol, or the pigment, ordinarily, a polymer which aids dispersion and whose viscosity is low and a plasticizing agent are prescribed.

Among the various printing processes of the method for manufacturing in the present invention, in a first printing process, instead of the ink in the conventional ink jet printing, the above-described solution or dispersion liquid of the drug is used and the drug is ink jet printed to a surface of a tablet or a capsule as a base. As the drug solution or dispersion liquid used in the first printing process, there are a dye type in which a drug is dissolved in the solvent, instead of the ink dye, and a pigment type in which a drug which is made fine is dispersed in a dispersion medium, instead of the pigment.

In a case where a dye type solution (drug solution) of a drug is used as the ink and the drug is ink jet-printed, when the drug liquid is applied to the surface of the tablet, the solvent and the drug are slightly soaked inside the tablet and the solvent is evaporated, thereby completing the ink jet printing of the drug to the surface of the tablet or the capsule.

In a case where the drug is not dissolved in the solvent, a pigment type dispersion liquid (drug dispersion liquid) of he drug is used as the ink. The drug dispersion liquid can be prepared by making the drug fine by employing various methods and by imparting an increased viscosity by a polymer so as to hardly precipitate the drug dispersion liquid.

In addition, although kinds of ink are differently used depending on a purpose of an object to be printed in ordinary printing onto a paper surface, in the printing onto the tablet, in view of clearness of printing, a pigment type ink is mainly used.

In any of the drug solution and the drug dispersion liquid, since the surface tension exerts influence, a surfactant agent can also be added. If the surface tension of the drug solution or the drug dispersion liquid is low, a spherical shape can be hardly obtained, and if the surface tension thereof is excessively high, a fine particulate spherical shape results, and digit-control to a constant amount cannot be performed.

Although as an applied drug, a drug which is highly active and whose content is small (approximately 10 mg or less) is suited, when the drug is dissolved by applying prescription which is similar to the prescription of the ink (liquid properties are suitable for the ink jet), a predetermined amount can be sprayed by the ink jet method and applied to one tablet. This amount depends on the prescription or properties of the drug.

Although in the ordinary prescription, a prescribed amount of the dye or the pigment is approximately 15% or less of the liquid, consideration is required such that liquid properties of the dissolubility of the drug and a viscosity and surface tension of a prepared liquid, which are suited for the ink jet method, can be retained.

In a case where a drug used in the present invention are not dissolved in water, alcohol, or a mixed liquid of the water and the alcohol, as with the case of the pigment, the drug is mechanically made fine to be dispersed, and it is required to process the drug such that the drug is not precipitated during the ink jet printing. Since steroid and active vitamin Ds are hardly soluble in the water, it is required to sufficiently consider drug liquid prescription used instead of the ink.

Although in the pigment type ink prescription, making the pigment fine is an important point of the ink preparation, by employing high-speed injection from a fine flow passage at a high pressure, a wet crushing method for generating nanoparticles to be ejected, a bead mill using minute beads (MICROFLUIDIZER, JET MILL, a wet bead mill), and the like, processing is performed such that a size in D90 is approximately 0.2 µm (200 nm) or less. However, although this value is a value as to each of ordinary ink jet nozzles, which has 600 dpi, this value depends on a bore diameter of each of the nozzles. Accordingly, by making a nozzle diameter large, even when each coarse particle size of 0.2 µm or more in D90 can be sprayed and applied by securing liquid properties which do not allow precipitation.

Although the dye type ink is widely used in ordinary printing to paper, such dye type ink is poor in light resistance and further, adhesion properties of the ink sprayed in a surface state of a printed target change. In addition, since in a surface of an orally disintegrating tablet having properties of easily disintegrating in water, a liquid is easily soaked, and bleeding of printed characters tends to be caused, the pigment type ink is mainly used in an ordinary ink jet type printing machine. Since in a dye type ink composition, coloring matter is dissolved in an ink liquid, little precipitation and separation are caused in a container or on a liquid line, and few troubles stemming from these factors are caused. Furthermore, although diverse colors can be represented by mixing kinds of coloring matter of red, blue, and yellow colors, the dye type ink composition has a drawback that color fading is caused by light.

Since in a case of the pigment type ink, water is easily soaked in the surface of the tablet in general, the pigment remains in the surface thereof, thereby resulting in a brilliant printing state. Since in the present invention, as the drug liquid for the ink jet, the dye type or pigment type drug liquid is selected depending on properties of the drug, both prescription systems are taken into consideration to prescribe the drug liquid to be used.

When a formulation is manufactured by employing the method of the present invention, as usable polymers, not only polymers used in the conventional film coating but also any polymers may be used as long as a viscosity (0.5 mPa·s to 200 mPa·s) and surface tension (30 mN/m to 80 mN/m) as to the liquid for dispensing for the ink jet printing can be ensured. Needless to say, these physical properties also depend on a nozzle diameter and a number of nozzles.

In addition, usable targets are different depending on a state of the surface of the tablet or capsule targeted for the ink jet printing.

When a drug solution is colorless and transparent or a dispersed small-amount drug is white, problems due to color are not caused upon spraying the drug solution to a white tablet. In addition, influence such as bleeding does not remarkably appear. However, since discoloring of a sprayed portion may be caused depending on temporal stability, a temperature and a humidity and light stability of a drug should be previously investigated.

As described above, in the method in which the drug-containing liquid is applied to the tablet or capsule by the ink jet printing technology, in any of the cases of the piezo type or the thermal type, physical properties and an application amount (several pL to several tens nL) of the liquid are limited. However, this method is a method which allows an extremely small amount of a substance to be applied with high accuracy.

Since in a case where a drug-containing liquid or a suspension dispersion liquid is applied by the dispensing method, a drug particle size and the like do not pose any problem, it is not really required to conduct filtering. However, it is required to filter the drug-containing liquid or the like applied to the ink jet printing by a filter in a structure of an application system and to prevent clogging inside an apparatus. As a filter used for the liquid, ordinarily, a filter having a bore diameter of 0.6 µm is used, and although a situation varies depending on a bore diameter of each of nozzles, in order to avoid troubles such as precipitation of the pigment and clogging of the nozzle head on the liquid line, attention should be paid to a particle diameter of the pigment. Pulverization of the pigment is conducted by the high-speed injection at a high pressure from a fine flow passage, the wet crushing method for generating nanoparticles to be ejected, the bead mill (MICROFLUIDIZER, JET MILL, the wet bead mill) using the minute beads, or the like. Since a preferable particle diameter after the pulverization is 200 nm or less in D90, processing is performed by a filter having this bore diameter.

In addition, in the case of the ink jet printing, in a case where the drug is dissolved in only water or a water-organic solvent system as a solvent used by the drug, such a solution can be used for the ink jet printing as it is. However, even in such a case, in order to prevent clogging at the nozzle head, it is preferable that the solution is filtered by a membrane filter having a bore diameter of 0.6 µm and thereafter, the filtered solution is used.

In a case where the drug is hardly soluble and is not dissolved in the water, the water-organic solvent system, and the organic solvent system as the solvent, it is required to make the drug fine by the above-mentioned bead mill or the like. As with the case of the solution, before being used for the ink jet, the obtained dispersion liquid is filtered by the membrane filter having the bore diameter of 0.6 µm and thereafter, is used.

In the case of the ink jet printing, in the drug solution or the drug dispersion liquid sprayed by the ink jet method, the water, the organic solvent (having a large amount of ethanol), the polymer, the plasticizing agent, and the surfactant agent are prescribed. It is needed to check stability in a solution or dispersion liquid state and also stability of sprayed and dried mixed substances. In reality, it is required to check also compatibility with a tablet surface or tablet prescription (a raw material mixture).

The drug application technology described in the present invention is a technology in which a printing technology for tablets, electronic components such as printed circuit boards, display products, batteries, and further, printed matter such as ordinary paper is basically applied as a technology used for application to the tablet of the drug. This dispensing method has been applied in various fields such as application to biological experimental techniques and 3D printer technologies.

In the application step of the drug by the dispensing method, limitations of physical properties of the applied liquid and the like are different depending on whether any of the above-described methods is applied. In the dispensing method, various methods can be selected simply depending on a concentration, a viscosity, and the like of the liquid.

On the other hand, in the ink jet printing technology, kinds of ink having various colors, whose amounts are constant, are easily accurately sprayed and applied as colored points having constant sizes. Although at this time, important parameters are the viscosity and the surface tension of the ink solution or the dispersion liquid, performance of the apparatus is also important. Colors of the ink, sizes of digits, and a density of the points (dpi) are determined from the performance of the apparatus to some extent.

In an ordinary ink jet printing apparatus, colored points of 600 dpi (600 points per length of one inch (2.54 cm)) can be dotted. Since two-dimensional spaying is performed, 360,000 points can be sprayed and applied per square inch (645.16 mm²).

Although a size of one droplet varies depending on prescription or properties of the ink, an ordinary small size thereof is one pL to 2 pL (picoliter: 1/1,000,000 of one mL) and a large size thereof is several tens pL. Here, it is assumed that the ink jet is performed with 10 pL and that the points of 600 dpi can be dotted, in printing to flat tablets each having a diameter of 8 mm, 27,900 points of the liquid each having 10 pL can be sprayed in an area of 50 mm² (in a round shape having eight mm) by this ink jet printing method. In other words, droplets of a liquid each having a size of 279 nL can be sprayed. If it is considered that a drug concentration of this liquid is 10% by weight, a drug of 27.9 µg is sprayed per one tablet.

It is seen from these calculations that a drug in a µg unit can be easily sprayed and applied per tablet. However, for a liquid whose application amount is slightly large or a liquid whose solubility is low, processing can be performed by increasing a concentration of a sprayed liquid or adjusting a sprayed application amount. Furthermore, in such a case, by adjusting spraying conditions of the ink jet (a nozzle diameter, a voltage, and the like), a droplet size and a number of droplets can be freely controlled, thereby allowing a wide range of sprayed application amounts to be ensured.

Furthermore, in the method in which the drug is applied by this ink jet method, a series of spraying application steps in the ink jet method can be performed as a plurality of steps, or several droplets of the same drug liquid can be sprayed and applied once. In addition, a different drug having poor compatibility is sprayed and applied to one tablet as another point or surface, and separation and application are performed, thereby preparing a stable compounding agent. Furthermore, in order to avoid problems such as exfoliation and peeling-off of the drug from the tablet, an overcoat can be applied.

As described above, it is also seen that by employing the method for manufacturing for manufacturing the tablets or the capsules by utilizing the dispensing method described in the present invention, some new advantages which have been impossible in the conventional manufacturing method (containment of a small amount of an active drug, a drug handling method such as application of a plurality of drugs, and an advanced spraying and coating method such as multiple coating) can be simultaneously attained.

When a drug having high activity is handled in general, in order not to scatter and diffuse the drug to the outside, manufacturing is conducted in a containment (confinement) handling manner. Basically, a working room is completely shielded from the outside, and working under a negative pressure environment (like an environment in which workers are in a vacuum cleaner) is forced. In particular, since there is a concern that a drug such as steroid exerts influence on female workers, it is required to strictly manage selection of workers and working hours (with also the above-mentioned hardware included).

In the dispensing method in the method of the present invention, the drug is handled as the solution or the suspension dispersion liquid and is used in manufacturing, thereby obtaining a predetermined formulation. In the process of preparing the liquid, the scattering and the like of the drug can be controlled, thereby making small the concern that workers are exposed to the drug.

By handling the drug by the method of the present invention, it is not required to make specifications of complete containment (confinement) for equipment having a large scale, in which granulation, mixing, solidification, coating, and the like are conducted, and handling which has few problems in terms of safety is enabled. Specifically, it is only required to conduct application and coating of the drug to the surface of each of the tablets or the capsules which does not contain a drug, which are prepared in ordinary equipment by a dispensing apparatus like a closed system-ink jet printing machine, thereby making the application and coating efficient. In addition, since by bringing a module of the dispensing apparatus in a slightly negative pressure or conducting local ventilation, diffusion of the small-amount drug is suppressed and further, a structure of the machine which allows a part of the machine to be detached and be thoroughly cleaned can be easily obtained, thereby making small a danger that workers are exposed to the drug.

It is required to manufacture a formulation having a large drug content by a large-scale facility for drugs having high activity. However, in the method for manufacturing the formulation containing the small-amount drug in the present invention, contacting the drug can be made in a small space such as a tablet printing apparatus or an inspection apparatus, thereby easily conducting the containment.

As described above, the pharmaceutical preparation is manufactured by the method for manufacturing the pharmaceutical preparation according to the present invention, thereby allowing the scattering of the drug to be prevented.

As described above, in the method for manufacturing according to the present invention, it is preferable that the first application step is conducted in the closed system.

In addition, in the first application step, as in ink jet type printing in which many nozzles as applied to a tablet printing machine are arranged in one row, ink of a several pL level is sprayed, and a drawing pattern is controlled by a computer or as in ink jet type printing in which a slightly larger amount of a solution or a dispersion liquid can be sprayed, wide selection of a nozzle diameter, a number of nozzles, a liquid viscosity, and the like can be made so as to allow dropping of various amounts. In addition, an advantage of this method is that many droplets having high accuracy can be applied to a prescribed area with high accuracy. Accordingly, since the system can spray the solution or the dispersion liquid with high accuracy and is optimum for application of a constant amount of a drug or the like to a formulation, and content uniformity of the formulation is strictly secured.

In the dispensing method other than the ink jet printing method, a wide variety of options as to a liquid viscosity and an application amount are available depending on a number of nozzles and an application method. However, in a case of application of a small amount, the ink jet printing method in which a constant amount of an extremely small amount is sprayed and applied with high accuracy is advantageous in terms of accuracy.

Furthermore, since on a surface of the formulation (tablets or capsules) obtained in the present invention, the drug is unevenly present, a formulation which is easily eluted in a hardly soluble drug can be obtained.

In addition, in a case where a drug is hardly soluble, when the drug is applied to a formulation together with a certain kind of a polymer, the drug which is in a non-crystalline form as a solid dispersion can also be applied to the surface of the formulation, thereby allowing elution properties to be improved.

In a case of the conventional method for manufacturing in which the drug is diluted by an excipient and is present as a solid preparation, in a formulation whose drug content is small in particular, as a phenomenon which is ordinarily observed in a formulation study, since influence of the excipient or the like is relatively large, the formulation becomes instable. In a case where the formulation is prepared by employing the method of the present invention and unevenly applying, to formulation, the drug in a state in which the drug is mixed with a small amount of the excipient, exposure of the drug to the excipient can be suppressed, thereby favorably leading to stabilization.

In addition, the method for manufacturing according to the present invention may include a coating step of coating the drug applied by the dispensing method in the first application step.

By employing the method for manufacturing the small-amount-drug containing formulation according to the present invention, not only properties of sustained-release effect, enteric effect, masking effect, and the like which the dissolved polymer additive has can be imparted to some extent, but also in the coating step, an overcoat such as an easily soluble polymer can be applied to the same places of the formulation (tablets) and processes in which falling-off and diffusion of the drug which is initially accurately applied in a constant amount can be simultaneously performed.

In addition, the method for manufacturing according to the present invention includes a second application step in which a second drug which is different from the drug applied in the first application step is applied to the surface of the tablet or the capsule by the dispensing method, and it is preferable that the second drug applied by the dispensing method in the second application step is applied in such a way as to be separated from the drug applied by the dispensing method in the first application step.

In this way, since contacting of the drug applied in the first application step and the second drug can be reduced, even in a case where compatibility of the drug applied in the first application step and the second drug is bad, stability of the drug can be enhanced.

As described above, by applying the techniques proposed in the present invention for manufacturing of the small-amount-drug containing formulation, many problems which are difficult to be solved by the conventional technology can be overcome.

As described above, as the method for applying the small-amount of the drug to the formulation such as the tablets, a new method for manufacturing in which the dispensing method is applied has been found. It is made possible to manufacture the small-amount-drug containing formulation without conducting extra manipulations which have been conducted in the blending process and the like in the conventional manufacturing processes of the formulation. In addition, by applying the method for manufacturing proposed in the present invention, the new method for manufacturing the formulation which allows safe handling of the drug having high activity, which has been impossible in the conventional method, and allows additional values to be imparted to the formulation can be obtained.

The present invention is summarized as described below.
(1) The method for manufacturing the pharmaceutical preparation according to the present invention includes: using the liquid for dispensing, the liquid contains the drug; the first application step of applying the drug to the surface of the tablet or capsule by the dispensing method.
(2) In the method for manufacturing the pharmaceutical preparation according to the present invention, it is preferable that the first application step is a process in which the drug is printed by the ink jet printing as the dispensing method.
(3) It is preferable that the method for manufacturing according to the present invention includes the coating step of coating the applied drug in the first application step.
(4) It is preferable that in the method for manufacturing according to the present invention, an amount of the applied drug in the first application step is 15 mg or less.
(5) In the method for manufacturing the pharmaceutical preparation according to the present invention, it is preferable that the second application step of applying the second drug or the excipient to the surface of the tablet or the capsule by the dispensing method is further included, the second drug being different from the drug applied in the first application step, and the second drug or the excipient applied in the second application step is printed in such a way as to be separated from the drug applied in the first application step.
(6) In the method for manufacturing the pharmaceutical preparation according to the present invention, it is preferable that the second application step is a process in which the second drug or the excipient is printed by ink jet printing as the dispensing method.
(7) In the method for manufacturing the pharmaceutical preparation according to the present invention, it is preferable that the first application step is conducted in the closed system.
(8) In the method for manufacturing the pharmaceutical preparation according to the present invention, it is preferable that the application area of the drug applied in the first application step is determined based on the amount of the drug contained in the pharmaceutical preparation.
(9) In the method for manufacturing the pharmaceutical preparation according to the present invention, it is preferable that relative standard deviation (RSD) of a content of the drug applied in the first application step is less than 3.8%.
(10) In the method for manufacturing the pharmaceutical preparation according to the present invention, it is preferable that the category of occupational exposure limits (OEL) of the drug applied in the first application step or the second drug applied in the second application step is 4 or more.
(11) The method for uniformizing a drug content in a pharmaceutical preparation includes a step of manufacturing the pharmaceutical preparation by the method for manufacturing the pharmaceutical preparation according to any one of the above-mentioned methods for manufacturing the pharmaceutical preparation.
(12) The method for preventing the scattering of the drug in manufacturing of the pharmaceutical preparation includes the step of manufacturing the pharmaceutical preparation by the methods for manufacturing the pharmaceutical preparation according to any one of the above-mentioned methods for manufacturing the pharmaceutical preparation.
(13) The pharmaceutical preparation includes: the base; and the drug applied to the surface of the base by the dispensing method, and the base is the tablet or the capsule.
(14) It is preferable that in the pharmaceutical preparation according to the present invention, the drug is the drug printed by the ink jet printing as the dispensing method.
(15) In the pharmaceutical preparation according to the present invention, it is preferable that the drug is a drug printed by ink jet printing as the dispensing method.
(16) In the pharmaceutical preparation according to the present invention, it is preferable that the amount of the drug applied by the dispensing method is 15 mg or less.
(17) In the pharmaceutical preparation according to the present invention, it is preferable that the second drug or the excipient is included, the second drug applied to the surface of the base by a dispensing method in such a way as to be separated from the drug applied by the dispensing method, the second drug being different from the drug.
(18) In the pharmaceutical preparation according to the present invention, it is preferable that the second drug or the excipient is a drug printed by ink jet printing as the dispensing method.
(19) In the pharmaceutical preparation according to the present invention, it is preferable that the relative standard deviation (RSD) of the content of the drug applied by the dispensing method is less than 3.8%.
(20) In the pharmaceutical preparation according to the present invention, it is preferable that each of the categories and/or the category of the occupational exposure limits (OEL) of the drug and/or the second drug is 4 or more.

### [Examples]

Hereinafter, although the present invention is further specifically shown by Examples, the present invention is not limited to these Examples.

### [Examples 1 to 7]

### Preparation of Raw Tablets for Application of Drug

The following two kinds of raw tablets (the conventional tablet and an orally disintegrating tablet), in each of which ordinary generally used raw materials are blended, were prepared, and application of a simulant drug by a dispensing method and evaluation of content uniformity were conducted.

### Conventional Tablet (F1):

Powder obtained by mixing 2,970g of granulated lactose hydrate (Dilactose (registered trademark) F, manufactured by Freund Corporation) and 30g of magnesium stearate (manufactured by TAIHEI CHEMICAL INDUSTRIAL CO., LTD.: vegetable) in a polyethylene bag was tableted by using a rotary tablet press (VIRGO manufactured by KIKUSUI SEISAKUSHO LTD.). As to a size of a tablet, a corner angle flat tablet having a diameter of 8.0 mm, a corner angle flat tablet having a diameter of 8.0 mm, and a go stone tablet having R1.2 mm were prepared, and each one tablet has 240 mg. In addition, corner angle flat tablets having a diameter of 10.0 mm and a diameter of 13.0 mm, whose weights were 400 mg and 500 mg, were also prepared.

### Orally Disintegrating Tablet (OD tablet) (F2):

Powder obtained mixing 2,970g of granulated D-mannitol (Granutol (registered trademark) R, manufactured by Freund Corporation) and 30g of magnesium stearate (manufactured by TAIHEI CHEMICAL INDUSTRIAL CO., LTD.: vegetable) in a polyethylene bag was tableted by using a rotary tablet press (VIRGO manufactured by KIKUSUI SEISAKUSHO LTD.). Sizes of tablets in Examples were different from one another, and as shown in Table 1, corner angle flat tablets each having a diameter of 8.0 mm, whose weight per tablet was 240 mg, were prepared.

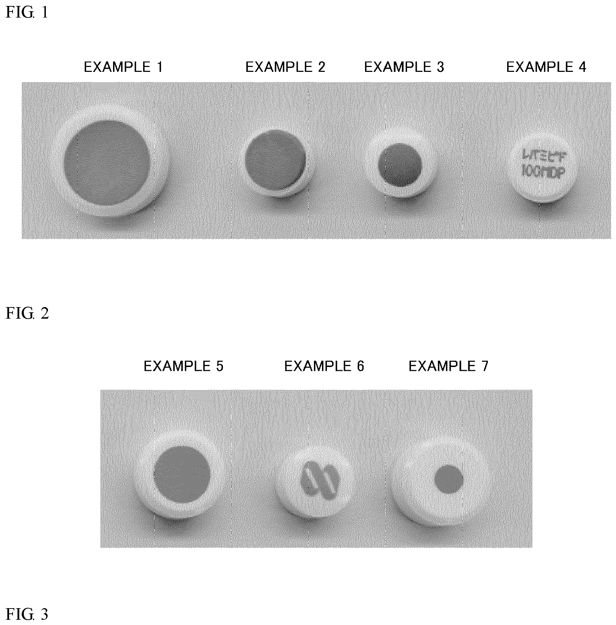

### Dye which contained 2.0% by weight of coloring matter of Food Blue No. 1 (Brilliant Blue) as an alternative of a drug (simulant drug) and 3.0% by weight of hypromellose as a binder was ink jet-printed to a surface of each of these tablets. In Examples 1 to 4, by using green dye type ink (coloring matter was a liquid (having a viscosity of approximately 5 mPa·s)) in which Food Blue No. 1, Food Yellow No. 4: Tartrazine, and Food Red No. 102 were blended at a ratio of 2 : 2 : 1), tablet surfaces of the respective tablets shown in Table 1 were subjected to printing whose printing areas and printing shapes were different from one another by a compact ink jet printing machine (IIM-Labo manufactured by MATSUOKA MACHINERY WORKS CO.,LTD) which includes an ink jet head of 600 dpi. In addition, in Examples 5 to 7, by using blue dye type ink (2% by weight of Food Blue No. 1)(having a viscosity of approximately 5 mPa·s), three kinds of printing whose printing areas and printing shapes were different from one another were performed by a compact ink jet type tablet printing machine (DP-i1000) manufactured by SCREEN Holdings Co., Ltd. which was equipped with an ink jet head of 600 dpi. Photographs of the tablets in Examples are shown in FIGS. 1 and 2.

**[Table 1]**

| | Tablet | | | |
|---|---|---|---|---|
| | Kind | Shape | Color | Printing Area (mm²) |
| Example 1 | Conventional (F1) | Flat Tablet having 13 mmcp | Green | 78.5 |
| Example 2 | OD (F2) | Flat Tablet Having 8 mmϕ | Green | 38.5 |
| Example 3 | Conventional (F1) | Go Stone Tablet Having R8 mmcp | Green | 19.6 |
| Example 4 | Conventional (F1) | Flat Tablet Having 8 mmϕ | Green (Characters) | 7.1 |
| Example 5 | Conventional (F1) | Flat Tablet Having 10 mmϕ | Blue | 30.2 |
| Example 6 | Conventional (F1) | Go Stone Tablet Having 8 mmcp | Blue (Logo) | 10.3 |
| Example 7 | Conventional (F1) | Flat Tablet Having 10 mmϕ | Blue | 7.1 |

### Evaluation Method of Contents of Simulant Drugs

Ten tablets among printed tablets obtained in each of Examples were taken; the ten tablets were inputted into a measuring flask having a capacity of 5 mL one by one; five mL of an extraction solvent (water : acetonitrile = 3 : 1) was added thereto; and each of the ten tablets was disintegrated by ultrasonic processing. Filtration was performed by a filter having a bore diameter of 0.45 µm; initial 2 mL was discarded; the remaining 3 mL was used as a specimen. Fifty µL of an ammonium phosphate buffer solution (pH7.3) having a concentration of 20 mM : acetonitrile = 3 : 1 liquid was injected as a mobile phase into an ODS column (40°C) having 4.6 mm x 150 mm and 5 µm of high-performance liquid chromatograph; a liquid velocity was adjusted such that retention time of a peak of Brilliant Blue was four minutes; and detection was conducted with 600 nm. A standard solution was prepared by dissolving 20 mg of Brilliant Blue in 100 mL of an extraction solvent and diluting 0.5 mL of the resultant to 50 mL. Quantitative values were calculated from area values. Results are shown in Tables 2 and 3.

**[Table 2]**

| | Content (µg) of Blue No. 1 (Brilliant Blue) | | | |
|---|---|---|---|---|
| | Example 1 | Example 2 | Example 3 | Example 4 |
| Printing Area (mm²) | 78.5 | 38.5 | 19.6 | 7.07 |
| No.1 | 4.63 | 2.13 | 1.15 | 0.33 |
| No.2 | 4.53 | 2.23 | 1.12 | 0.34 |
| No.3 | 4.62 | 2.13 | 1.13 | 0.35 |
| No.4 | 4.55 | 2.23 | 1.14 | 0.33 |
| No.5 | 4.54 | 2.18 | 1.13 | 0.33 |
| No.6 | 4.56 | 2.16 | 1.11 | 0.33 |
| No.7 | 4.50 | 2.07 | 1.13 | 0.34 |
| No.8 | 4.46 | 2.19 | 1.16 | 0.33 |
| No.9 | 4.49 | 2.21 | 1.16 | 0.34 |
| No.10 | 4.52 | 2.19 | 1.14 | 0.32 |
| Average | 4.54 | 2.17 | 1.14 | 0.33 |
| RSD (%) | 1.2 | 2.3 | 1.6 | 2.3 |

**[Table 3]**

| | Content (µg) of Blue No. 1 (Brilliant Blue) | | |
|---|---|---|---|
| | Example 5 | Example 6 | Example 7 |
| Printing Area (mm²) | 30.2 | 10.3 | 7.1 |
| No.1 | 6.17 | 2.00 | 1.43 |
| No.2 | 6.26 | 2.04 | 1.49 |
| No.3 | 5.84 | 1.90 | 1.45 |
| No.4 | 6.31 | 1.93 | 1.48 |
| No.5 | 5.98 | 2.08 | 1.50 |
| No.6 | 6.05 | 2.03 | 1.44 |
| No.7 | 5.88 | 2.07 | 1.49 |
| No.8 | 6.12 | 2.11 | 1.45 |
| No.9 | 6.19 | 2.04 | 1.42 |
| No.10 | 6.02 | 2.07 | 1.46 |
| Average | 6.08 | 2.02 | 1.49 |
| RSD (%) | 2.6 | 3.3 | 1.9 |

Variation in the contents of the drugs printed in round forms was small and favorable results were obtained. Although it is considered that variation in the tablets printed with the characters or in a figure shape was large due to the complicated shapes, it is likely that in a case of the DP-i1000 manufactured by SCREEN Holdings Co., Ltd., printing unevenness was caused due to properties of the apparatus, and the variation was slightly large. In any case, it was seen that the small-amount drug can be applied by the ink jet technology with little variation.

An area of a printed part was estimated by directly measuring a diameter of a round form, and as to the characters and the logo, a rough area was calculated by ImaWorks which is a contour measurement software. Relationship between a content (µg) of the ink sprayed and applied from the ink jet nozzle head and a printing area was plotted. As shown in FIG. 3, the printing area of the ink and the coloring matter amount were proportional to each other. In addition, in these seven Examples, relative standard deviation (RSD %) was 1.2 to 3.3. It was found that in each of respective apparatus-ink groups, a straight line passing through a point of origin resulted and an amount which is proportional to a content rate was applied. Since also in a case where the apparatus and the concentration of the ink were changed, in the same manner, relationship of the sprayed and applied area and the dye was clear, it was considered that it was made possible to ensure the quantitative value with considerable accuracy. Accordingly, it was considered that also in a case where instead of the ink, a drug solution was used, it was made possible to ensure similar accuracy.

Accordingly, in the method for manufacturing of the pharmaceutical preparation according to the present invention, it is preferable that in the first application step, the printing areas of the drug subjected to the ink jet printing are determined based on the amounts of the drug contained in the pharmaceutical preparation.

### [Comparative Examples 1 to 3]

As with the Examples, as a simulant drug, Food Blue No. 1 (Brilliant Blue) was used, and coloring matter excipient-diluted granules in prescription shown in Table 4 were prepared.

In Comparative Example 1, coloring matter (a simulant drug) was added to a binder solution in the prescription shown in Table 4 and the resultant was granulated, thereby obtaining coloring matter excipient-diluted granules. These granules were mixed with the Dilactose formulation of F1 used in Examples, and the resultant was sufficiently mixed such that a total amount thereof with magnesium stearate contained was 100 g and was tableted. A concentration of the coloring matter (simulant drug) of the coloring matter excipient-diluted granules was 0.36%.

In Comparative Example 2, granules obtained by diluting the coloring matter (Brilliant Blue) directly with the Dilactose F were used as coloring matter excipient-diluted granules, and the remaining was mixed with a F1 formulation (Dilactose F + magnesium stearate), thereby obtaining 100g of tableted granules.

In Comparative Example 3, water was added to the coloring matter excipient-diluted granules prepared in Comparative Example 2, and granules in which coloring matter was dispersed were subjected to drying and size regulation, thereby obtaining coloring matter excipient-diluted granules, and 56 mg to 556 mg of the coloring matter excipient-diluted granules were mixed with the F1 formulation to obtain tableted granules, thereby preparing tablets. In any of Comparative Examples, the mixing was manually performed, and the mixing was sufficiently performed.

**[Table 4]**

| Raw Material | | Comparative Example 1 | Comparative Example 2 | Comparative Example 3 |
|---|---|---|---|---|
| Coloring Matter Excipient-Diluted Granules | Food Blue No. 1 | 72 mg | 24 mg | 12 mg |
| | Hypromellose | 0.700 g | - | - |
| | Crospovidone | 5.895 g | - | - |
| | Dilactose F | | 6.643 g | 6.655 g |
| Total of Coloring Matter Excipient-Diluted Granules | | 6.667 g | 6.667 g | 6.667 g |
| Charged Amount of Coloring Matter Excipient-Diluted Granules | | 56 mg to 556 mg | 56 mg to 556 mg | 56 mg to 556 mg |
| Dilactose F (99%) + Magnesium Stearate (1.0%) | | 100 g in Total | 100 g in Total | 100 g in Total |

The above-described coloring matter excipient-diluted granules were blended with 100g of the F1 formulation with the Dilactose F as a main ingredient, thereby preparing granules for tableting. In each of Comparative Examples, an amount of the coloring matter excipient-diluted granules blended to 100g of the F1 formulation was 56 mg to 556 mg. In other word, a blended amount in each of F1-001, F1-001-3, and F1-001-4 was 56 mg, a blended amount in each of F1-002, F1-002-3, and F1-002-4 was 111 mg, a blended amount in each of F1-003, F1-003-3, and F1-003-4 was 222 mg, and a blended amount in each of F1-004, F1-004-3, and F1-004-4 was 556 mg. The granules for tableting were tableted by a rotary tablet press (VIRGO manufactured by KIKUSUI SEISAKUSHO LTD.), thereby preparing corner angle flat tablets each having a diameter of 8.5 mm, whose each weight was 250 mg.

Analysis of contents of Blue No.1 of the tablets was conducted by the same method as in Examples. Results are shown in Tables 5 to 7.

**[Table 5]**

| | Content of Blue No. 1 (Brilliant Blue) (µg) | | | |
|---|---|---|---|---|
| | Comparative Example 1 F1-001 | Comparative Example 1 F 1-002 | Comparative Example 1 F 1-003 | Comparative Example 1 F1-004 |
| No.1 | 3.13 | 1.92 | 5.55 | 15.21 |
| No.2 | 1.41 | 1.64 | 10.89 | 13.77 |
| No.3 | 1.20 | 2.35 | 5.83 | 16.18 |
| No.4 | 1.12 | 1.74 | 9.32 | 12.07 |
| No.5 | 1.68 | 2.88 | 6.59 | 15.77 |
| No.6 | 1.56 | 2.52 | 4.29 | 14.61 |
| No.7 | 4.24 | 1.60 | 5.30 | 11.80 |
| No.8 | 2.73 | 1.58 | 5.04 | 13.04 |
| No.9 | 0.99 | 3.94 | 5.92 | 15.13 |
| No.10 | 0.84 | 3.40 | 5.17 | 17.90 |
| Average | 1.89 | 2.36 | 6.39 | 14.55 |
| SD | 1.11 | 0.83 | 2.08 | 1.93 |
| RSD (%) | 58.9 | 35.1 | 32.6 | 13.1 |

Variation in tablets in any of formulations in Comparative Example 1 was large. In particular, in a lot in which the contents were low, RSD approximated 60%.

**[Table 6]**

| | Content (µg) of Blue No. 1 (Brilliant Blue) | | | |
|---|---|---|---|---|
| | Comparative Example 2 F1-001-3 | Comparative Example 2 F 1-002-3 | Comparative Example 2 F1-003-3 | Comparative Example 2 F1-004-3 |
| No.1 | 0.33 | 0.58 | 1.33 | 4.49 |
| No.2 | 0.33 | 1.08 | 1.68 | 4.22 |
| No.3 | 0.40 | 0.67 | 2.60 | 3.28 |
| No.4 | 0.88 | 0.79 | 1.34 | 3.21 |
| No.5 | 0.83 | 0.65 | 1.24 | 4.59 |
| No.6 | 0.36 | 0.82 | 1.21 | 3.39 |
| No.7 | 0.54 | 0.66 | 1.70 | 3.52 |
| No.8 | 0.43 | 0.76 | 1.34 | 3.77 |
| No.9 | 0.35 | 0.91 | 1.46 | 3.43 |
| No.10 | 0.32 | 1.14 | 1.11 | 3.92 |
| Average | 0.48 | 0.81 | 1.50 | 3.78 |
| SD | 0.21 | 0.19 | 0.43 | 0.50 |
| RSD (%) | 44.1 | 23.2 | 28.6 | 13.3 |

As with Comparative Example 1, variation in tablets in any formulations in Comparative Example 2 was large.

**[Table 7]**

| | Content (µg) of Blue No. 1 (Brilliant Blue) | | | |
|---|---|---|---|---|
| | Comparative Example 3 F1-001-4 | Comparative Example 3 F 1-002-4 | Comparative Example 3 F1-003-4 | Comparative Example 3 F 1-004-4 |
| No.1 | 0.18 | 0.59 | 1.16 | 3.04 |
| No.2 | 0.14 | 0.48 | 1.41 | 3.02 |
| No.3 | 0.23 | 0.51 | 1.15 | 3.13 |
| No.4 | 0.25 | 0.54 | 1.04 | 3.02 |
| No.5 | 0.24 | 0.46 | 1.14 | 2.91 |
| No.6 | 0.17 | 0.49 | 1.22 | 3.01 |
| No.7 | 0.16 | 0.52 | 1.25 | 2.98 |
| No.8 | 0.14 | 0.45 | 1.08 | 2.96 |
| No.9 | 0.26 | 0.49 | 1.22 | 3.32 |
| No.10 | 0.18 | 0.43 | 1.05 | 2.98 |
| Average | 0.19 | 0.50 | 1.17 | 3.04 |
| SD | 0.05 | 0.05 | 0.11 | 0.12 |
| RSD (%) | 23.8 | 9.6 | 9.4 | 3.8 |

Tablets in Comparative Example 3 were prepared by sufficiently dispersing the coloring matter in the Dilactose F, adding water to the resultant, kneading the resultant, and drying the resultant to enhance dispersibility of the coloring matter. However, also in this case, variation was large. However, among these, variation in the formulation in which the contents were high was comparatively small.

As is seen from the results in Examples 1 to 7 and Comparative Examples 1 to 3, variation in contents in formulations (Examples 1 to 7) in which spraying and applying of the coloring matter were conducted by the ink jetmethod was small. In addition, relationship between the content and the application area in each of Examples 1 to 7 was accurate, and by specifying an area, it was made possible to prescribe an application amount. On the other hand, as in Comparative Examples 1 to 3, among the tablets prepared by ordinary manufacturing processes, variation in any of tablets prepared by comparatively simple direct tableting and tableting close to the direct tableting was large.

### [Examples 8 to 11]

In each of Examples 8 to 11, as described below in detail, a drug was applied to various raw tablets, the resultant was dried, and thereafter, trial tablets were obtained, and a quantitative test was conducted by using the trial tablets.

As a dispenser, an apparatus in which a high-speed desktop type robot, SHOTmini200SX (manufactured by Musashi Engineering, Inc.) was equipped with a contactless jet dispenser, AEROJET (manufactured by Musashi Engineering, Inc.) was used. This apparatus was charged with a drug-containing liquid, and a drug liquid was applied under various conditions and in a constant amount onto arrayed raw tablets, thereby preparing tablets. In any of Examples, a nozzle diameter of 19G was used.

As a drug, risperidone (3-{2-[4-(6-fluoro-1,2-benzisoxazole-3-yl) piperidine-1-yl] ethyl} -2-methyl-6,7,8,9-tetrahydro-4H-pyridio [1,2-a] pyrimidine-4-one) was used.

As raw tablets, two kinds of round tablets (M1) and tablets (F2) were used. The round tablets (M1) were prepared by adding 1% of magnesium stearate as a lubricant to SmartEx (granules granulated with mannitol, polyvinyl alcohol, and low substituted hydroxypropyl cellulose, manufactured by Shin-Etsu Chemical Co., Ltd.), mixing these to obtain granules, and tableting the granules each in 240 mg, each of the round tablets (M1) having 8 mmcp. The tablets (F2) were prepared by adding 1% of the lubricant as with the M1 to Granutol R (granulated mannitol) to obtain granules and tableting the granules to obtain tablets, each of the tables having the same size. In Examples 8 and 10, the round tablets (M1) were used, and in Examples 9 and 11, the tablets (F2) were used.

As a liquid for dispensing, an 80% ethanol-water mixed liquid containing 5% of hypromellose (TC-5R, manufactured by Shin-Etsu Chemical Co., Ltd.) which contained 1% of the drug was used. Note that a viscosity of this liquid for dispensing was approximately 50 mPa·s.

In Examples 8 to 9, approximately one mg per shot was applied, and in Examples 10 to 11, approximately 680 µg per shot was applied. In each of Examples, ten tablets were prepared.

Drug content measurement of formulations was conducted by employing an HPLC method. Results are shown in Table 8.

**[Table 8]**

| | Drug Content (µg) | | | |
|---|---|---|---|---|
| | Examples 8 | Examples 8 | Examples 8 | Examples 8 |
| | M1-One Shot | M1-One Shot | M1-One Shot | M1-One Shot |
| No.1 | 9.55 | 9.72 | 39.36 | 39.73 |
| No.2 | 9.63 | 9.86 | 39.27 | 38.87 |
| No.3 | 9.89 | 9.61 | 39.18 | 39.04 |
| No.4 | 9.71 | 9.81 | 38.77 | 38.86 |
| No.5 | 9.82 | 9.94 | 39.37 | 39.55 |
| No.6 | 9.67 | 9.73 | 39.24 | 38.96 |
| No.7 | 9.82 | 9.36 | 39.33 | 38.78 |
| No.8 | 9.62 | 9.76 | 39.17 | 39.39 |
| No.9 | 9.80 | 9.87 | 39.05 | 39.85 |
| No.10 | 9.64 | 9.75 | 39.25 | 39.37 |
| Average | 9.72 | 9.74 | 39.20 | 39.24 |
| SD | 0.11 | 0.16 | 0.18 | 0.39 |
| RSD(%) | 1.14 | 1.67 | 0.45 | 0.99 |

In any of Examples 8 to 11, results, in each of which variation with approximately 1% of RSD was small, were obtained, and it was seen that application of the drug was performed with high accuracy.

### [Examples 12 to 13]

In Examples 12 to 13, as described below in detail, a drug was applied to raw tablets, the resultant was dried, and thereafter, trial tablets were obtained, and a stability test was conducted by using the trial tablets.

As a dispenser, the same one as in Examples 8 to 11 was used.

As a drug, lubiprostone (7-[(2R, 4aR, 5R, 7aR)-2-(1,1-difluoropentane-1-yl)-2-hydroxy-6-oxooctahydrocyclopenta [b] pyran-5-yl] heptane acid) was used.

As raw tablets, two kinds of film-coated tablets (F3FC) and raw tablets (M3) were used. The film-coated tablets (F3FC) were prepared by spraying hydroxypropyl cellulose (HPC-L) to crystalline cellulose, granulating the resultant to obtain granules, adding 1% of magnesium stearate (vegetable) as a lubricant to the granules, tableting the granules to obtain tablets, and film-coating 5 mg of hypromellose to the tablets (a weight of 220 mg) having 8 mmcp and R1.2 mm. The raw tablets (M3) were prepared by mixing crospovidone (XL-10) to crystalline cellulose at a mixing ratio of 30% of the crospovidone and 70% of the crystalline cellulose to obtain granules, lubricating the granules by magnesium stearate, and tableting the granules each having a weight of 130 mg.

Prescription of a liquid for dispensing was as shown in Table 9.

**[Table 9]**

| Raw Material | Prescribed Amount |
|---|---|
| Lubiprostone | 90 mg |
| Hypromellose (TC-5R) | 2.91 g |
| Ethanol | 21.6 g |
| Purified Water | 5.4 g |
| Total | 30.0 g |

In Example 12, two shots of the liquid for dispensing (a viscosity was approximately 200 mPa·s) prepared in the prescription shown in Table 9 were applied onto the film-coated tablets (F3FC), with approximately 720 µg of the liquid for dispensing per shot, and in Example 13, five shots thereof were applied onto the raw tablets (M3), with approximately 720 µg of the liquid for dispensing per shot. In each of Examples, five tablets were prepared.

Harshness stability (at 60°C and RH of 75%) of the obtained tablets was checked. A constant amount was measured by using LC-MS/MS (Acquity H-Class UPLC system, coupled to a Xevo TQ-S Micro Tandem Mass Spectrometer, Waters) and employing an internal standard method. Results are shown in Tables 10 to 11.

**[Table 10]**

| | Drug Content (µg) | | |
|---|---|---|---|
| Formulation | Initial | 60°C and RH of 75% after Two Weeks | Residual Rate (%) after Two Weeks |
| No.1 | 4.32 | 4.15 | 96.0 |
| No.2 | 4.32 | 4.10 | 94.9 |
| No.3 | 4.35 | 4.32 | 100.1 |
| No.4 | 4.28 | 4.35 | 100.6 |
| No.5 | 4.32 | 4.00 | 92.7 |
| Average | 4.32 | 4.18 | 96.9 |

**[Table 11]**

| | Drug Content (µg) | | |
|---|---|---|---|
| Formulation | Initial | 60°C and RH of 75% after Two Weeks | Residual Rate (%) after Two Weeks |
| No.1 | 10.16 | 10.69 | 98.1 |
| No.2 | 10.98 | 10.75 | 98.6 |
| No.3 | 11.12 | 11.16 | 102.4 |
| No.4 | 10.89 | 11.08 | 101.7 |
| No.5 | 11.34 | 10.73 | 98.4 |
| Average | 10.90 | 10.88 | 99.9 |

As shown in Tables 10 to 11, it was shown that even after two weeks, a residual rate was high and stability was exhibited. In particular, in Example 13, although the raw tablets (M3) including the crystalline cellulose whose compatibility with the drug substance was poor were used, stability even after two weeks as a harshness condition was exhibited.

### [Comparative Example 4]

A binder liquid was prepared by dissolving lubiprostone as a drug in an 80%-ethanol-water solution in which hypromellose was dissolved; the binder liquid was added to a mixed power constituted of a lactose hydrate, corn starch, crystalline cellulose, and crospovidone; the resultant was granulated in a high-speed mixing granulator (VG-01: manufactured by Powrex Corporation) to obtain granules; the granules were dried and were subjected to size regulation by a tumbling fluid bed dryer (MP-01: manufactured by Powrex Corporation); magnesium stearate was added to and mixed with the resultant granules for lubrication; and the resultant was tableted by a rotary tablet press (manufactured by VIRGO:KIKUSUI SEISAKUSHO LTD.). Contents in one tablet were as shown in Table 12.

**[Table 12]**

| Components | Contents in One Tablet (mg) |
|---|---|
| Lubiprostone | 0.024 |
| Hypromellose | 10 |
| Lactose hydrate | 140 |
| Corn Starch | 48.8 |
| Crystalline Cellulose | 20 |
| Crospovidone | 10 |
| Magnesium Stearate | 1.2 |
| Total | 230.024 |

As to the obtained tablets, as with Examples 12 to 13, a harshness test at 60°C and RH of 75% after two weeks was conducted. As a result, a residual rate of the lubiprostone was reduced from the initial % to 79%.

It was shown from the results in Examples 12 to 13 and Comparative Example 4 that the tablets of the lubiprostone obtained by the method for manufacturing of the present invention were stable than the tablets manufacture by the conventional method.

### [Examples 14 to 15]

In Examples 14 to 15, stability was tested by changing an application amount of a drug.

As the drug, fingolimod (2-amino-2-[2-(4-octylphenyl) ethyl] propane-1,3-diol hydrochloride) was used.

As bases, the above-described F1, F2, M1, and M2 were used. A weight of any of those was 240 mg and each of those was a flat tablet having 8 mmcp.

As a dispenser, the same one as in Examples 8 to 11 was used.

In consideration of dissolubility of the fingolimod hydrochloride, a liquid for dispensing which was 5%-TC-5R in 90%-ethanol-water and whose viscosity was approximately 60 mPa·s was used.

In Example 14, as shown in Table 13, one shot, three shots, or five shots of the above-mentioned solution containing a 2.8% drug substance were applied, with 250 µg/one shot. In Example 15, a liquid containing a 2.8%-drug substance was applied, with approximately 15 µL per tablet.

### [Comparative Example 5]

As a drug, a fingolimod hydrochloride was used and was sufficiently mixed with lactose or mannitol as an excipient in concentrations shown in Table 15, thereby preparing powders.

A stability test (60°C and RH of 75% for three days in an open state) was conducted for the tablets in Examples 13 to 14 and the powders in Comparative Example 5. As an analysis method, an HPLC method was employed. Results are shown in Tables 13 to 15.

**[Table 13]**

| | Base: Drug Substance Concentration with a Predetermined Application Amount in ( ) | Drug Content (µg) | | Residual Rate (%) |
|---|---|---|---|---|
| | | Initial | 60°C and RH of 75% for Three Days in an Open State | |
| Example 14-1 | M2-1(6.8): 0.003% | 7.8 | 6.4 | 82 |
| Example 14-2 | M2-3(20.5): 0.009% | 21.6 | 20.5 | 95 |
| Example 14-3 | M2-5(34.2): 0.015% | 31.9 | 29.7 | 93 |
| Example 14-4 | F1-1(6.8): 0.003% | 6.1 | 4.5 | 74 |
| Example 14-5 | F1-3(20.5): 0.009% | 16.4 | 14.5 | 88 |
| Example 14-6 | F1-5(34.2): 0.015% | 22.8 | 22.7 | 99 |

**[Table 14]**

| | Base: Drug Substance Concentration with a Predetermined Application Amount in ( ) | Drug Content (µg) | | Residual Rate (%) |
|---|---|---|---|---|
| | | Initial | 60°C and RH of 75% for Three Days in an Open State | |
| Example 15-1 | F 1(420 µg) 0.17% | 460 | 454 | 98.6 |
| Example 15-2 | M1(420 µg) 0.18% | 457 | 452 | 98.7 |
| Example 15-3 | M2(420 µg) 0.18% | 456 | 441 | 96.8 |

**[Table 15]**

| | Excipient | Drug Content or Concentration | | | 60°C and RH of 75% for Three Days in an Open State Residual Rate (%) |
|---|---|---|---|---|---|
| | | Initial Concentration (%) | Initial Content (µg) | 60°C and RH of 75% for Three Days in an Open State Content (µg) | |
| Comparative Example 5-1 | Mannitol | 1.1 | 103.5 | 103.1 | 99.6 |
| Comparative Example 5-2 | Mannitol | 0.6 | 56.3 | 56.1 | 99.9 |
| Comparative Example 5-3 | Mannitol | 0.06 | 6.84 | 4.32 | 63.2 |
| Comparative Example 5-4 | Lactose | 1.0 | 108.6 | 103.1 | 94.9 |
| Comparative Example 5-5 | Lactose | 0.6 | 57.9 | 51.3 | 88.6 |
| Comparative Example 5-6 | Lactose | 0.05 | 6.47 | 3.68 | 56.8 |

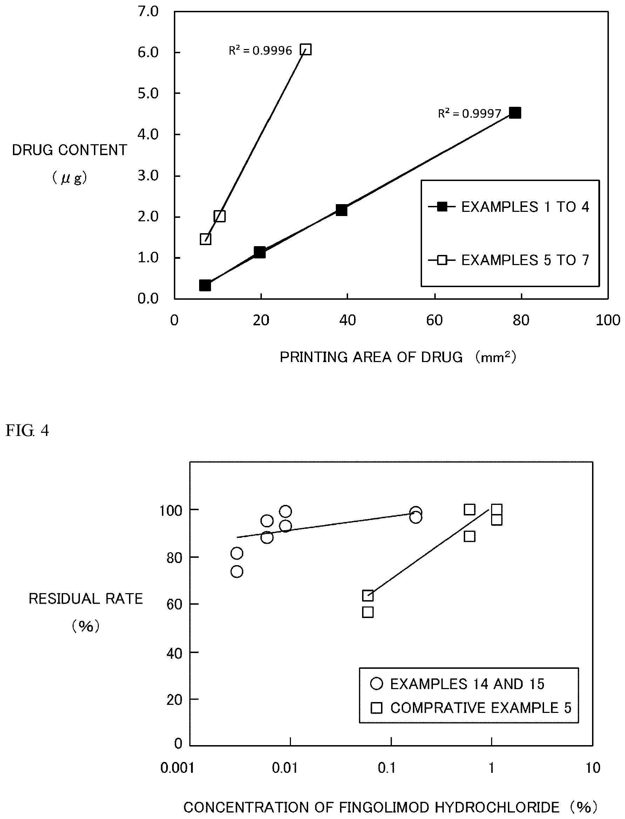

Results of stability of the formulation obtained by applying the fingolimod hydrochloride onto the tablets by dispensing (Examples 14 and 15) and results of stability of the pulverulent bodies in which the fingolimod hydrochloride and the excipient were mixed (Comparative Example 5) were plotted with a horizontal axis as a drug concentration in a logarithmic expression and a vertical axis as a residual rate (%), resulting in a graph in FIG. 4. Far better results of the stability of the tablets in Examples 14 to 15 than the results of the stability of the pulverulent bodies were obtained.

As shown in Tables 13 to 15, even in Comparative Example 5, in a case where the concentration of the drug was high, the stability was good. However, as shown in Examples 14 to 15, in a case where the method of the present invention was employed, even when the drug amount was small, the stability was high. In particular, in a case where the application amount was small (0.003% to 0.015% of a tablet weight) as in Example 14, the stability in a case where the application amount was large was higher as in Example 15. In addition, in Comparative Example 5, stability levels were different from one another depending on the kinds of the excipients, and in a case where the excipient was the lactose, more unstableness resulted than in a case where the excipient was the mannitol. However, in Examples 14 to 15, the kinds of the base (the lactose or the mannitol) did not exert large influence on the stability.

It was seen from the above-described results that according to the manufacturing method of the present invention, regardless of the application area of the drug, it was made possible to perform application to the tablet surface without large variation.

In addition, it was shown that it was made possible to apply the small amount of the drug to the tablets with accuracy. It was shown that it was made possible to apply the method of the present invention to manufacturing of the small-amount-drug containing formulation in reality. Furthermore, it was suggested that it was made possible to attain stabilization of the formulation manufactured by the manufacturing method of the present invention by localization of the drug.

In general, as to the small-amount-drug containing formulation, conventionally, in order to secure uniformity, mixing the drug and the excipient and processing the resultant in a formulation process such as granulation have been common practice. However, in the case of this manufacturing method, the drug was surrounded by the excipient and was influenced by the excipient, and in particular, in a case where compatibility between the excipient and the drug was bad, the stability has tended to be worsened. In the conventional method as described above, in a case where a content of the drug is small, the stability is further worsened. In addition, it has been common knowledge among formulation researchers that even as to a formulation of an ordinary dosage, when a content is low and a drug concentration is low, the lower a drug concentration is, the worse the stability is. In the manufacturing method of the present invention, since by applying the drug-containing liquid to the placebo tablets, the drug contacts only the small amount of the binder and the excipient on a placebo tablet surface and moreover, application can be performed at a high drug concentration, stabilization of the formulation can be made.

It was seen from these respects that the manufacturing method of the present invention can be applied to manufacturing of the small-amount-drug containing formulation in reality. In addition, a drug application step and the like can be conducted in a small space; further, also in production, a structure itself of a printing machine for production is a comparatively closed structure; and by slightly reworking the printing machine and making the printing machine to have a sealed structure in which drug scattering is hardly caused, a drug having high activity can be easily and safely handled, thus leading to great advantages.

As described above, by the method for manufacturing the pharmaceutical preparation of the present invention, a method for uniformizing a drug content in a pharmaceutical preparation can be provided. In addition, by the method for manufacturing the pharmaceutical preparation of the present invention, a method for preventing scattering of the drug in manufacturing the pharmaceutical preparation can be provided.

The described embodiment and Examples are to be considered in all respects only as illustrative and not restrictive. It is intended that the scope of the invention is, therefore, indicated by the appended claims rather than the foregoing description of the embodiment and Examples and that all modifications and variations coming within the meaning and equivalency range of the appended claims are embraced within their scope.

## Claims

1. A method for manufacturing a pharmaceutical preparation, comprising:
using a liquid for dispensing, the liquid containing a drug; and
a first application step of applying the drug to a surface of a tablet or a capsule by a dispensing method.

2. The method for manufacturing the pharmaceutical preparation according to claim 1, wherein the first application step is a process in which the drug is printed by ink jet printing as the dispensing method.

3. The method for manufacturing the pharmaceutical preparation according to claim 1 or 2, further comprising a coating step of coating the drug being applied in the first application step.

4. The method for manufacturing the pharmaceutical preparation according to any one of claims 1 to 3, wherein an amount of the drug being applied in the first application step is 15 mg or less.

5. The method for manufacturing the pharmaceutical preparation according to any one of claims 1 to 4, further comprising a second application step of applying a second drug or an excipient to a surface of a tablet or a capsule by a dispensing method, the second drug being different from the drug being applied in the first application step, wherein the second drug or the excipient being applied in the second application step is printed in such a way as to be separated from the drug being applied in the first application step.

6. The method for manufacturing the pharmaceutical preparation according to claim 5, wherein the second application step is a process in which the second drug or the excipient is printed by ink jet printing as the dispensing method.

7. The method for manufacturing the pharmaceutical preparation according to any one of claims 1 to 6, wherein the first application step is conducted in a closed system.

8. The method for manufacturing the pharmaceutical preparation according to any one of claims 1 to 7, wherein an application area of the drug being applied in the first application step is determined based on an amount of the drug being contained in the pharmaceutical preparation.

9. The method for manufacturing the pharmaceutical preparation according to any one of claims 1 to 8, wherein relative standard deviation (RSD) of a content of the drug being applied in the first application step is less than 3.8%.

10. The method for manufacturing the pharmaceutical preparation according to any one of claims 1 to 9, wherein a category of occupational exposure limits (OEL) of the drug being applied in the first application step or the second drug being applied in the second application step is 4 or more.

11. A method for uniformizing a drug content in a pharmaceutical preparation, comprising a step of manufacturing the pharmaceutical preparation by the method for manufacturing the pharmaceutical preparation according to any one of claims 1 to 10.

12. A method for preventing scattering of a drug in manufacturing of a pharmaceutical preparation, comprising a step of manufacturing the pharmaceutical preparation by the method for manufacturing the pharmaceutical preparation according to any one of claims 1 to 10.

13. A pharmaceutical preparation comprising:
a base; and
a drug being applied to a surface of the base by a dispensing method, wherein the base is a tablet or a capsule.

14. The pharmaceutical preparation according to claim 13, wherein the drug is a drug being printed by ink jet printing as the dispensing method.

15. The pharmaceutical preparation according to claim 13 or 14, further comprising coating which coats the drug being applied by the dispensing method.

16. The pharmaceutical preparation according to any one of claims 13 to 15, wherein an amount of the drug being applied by the dispensing method is 15 mg or less.

17. The pharmaceutical preparation according to any one of claims 13 to 16, further comprising a second drug or an excipient, the second drug being applied to a surface of the base by a dispensing method in such a way as to be separated from the drug being applied by the dispensing method, the second drug being different from the drug.

18. The pharmaceutical preparation according to claim 17, wherein the second drug or the excipient is a drug being printed by ink jet printing as the dispensing method.

19. The pharmaceutical preparation according to any one of claims 13 to 18, wherein relative standard deviation (RSD) of a content of the drug being applied by the dispensing method is less than 3.8%.

20. The pharmaceutical preparation according to any one of claims 13 to 19, wherein each of categories and/or a category of occupational exposure limits (OEL) of the drug and/or the second drug is 4 or more.
